# EUROPÄISCHE PATENTSCHRIFT

(11) **EP 1 085 926 B1**
(45) Veröffentlichungstag und Bekanntmachung des Hinweises auf die Patenterteilung: **08.10.2003**
(21) Anmeldenummer: 99929134.7
(22) Anmeldetag: 02.06.1999
(51) Int. Cl.: A61N 2/02

(54) **VORRICHTUNG ZUR MAGNETISCHEN STIMULATION EINES KÖRPERTEILS**
DEVICE FOR MAGNETICALLY STIMULATING A BODY PART
DISPOSITIF POUR LA STIMULATION MAGNETIQUE D'UNE PARTIE DU CORPS

(30) Priorität: 02.06.1998 DE 19824504
(43) Veröffentlichungstag der Anmeldung: 28.03.2001
(73) Patentinhaber: Struppler, Albrecht, 82340 Feldafing (DE); Havel, Peter, 93354 Biburg (DE)
(72) Erfinder: Struppler, Albrecht, 82340 Feldafing (DE); Havel, Peter, 93354 Biburg (DE)
(74) Vertreter: Heselberger, Johannes
(86) Internationale Anmeldenummer: EP9903851
(87) Internationale Veröffentlichungsnummer: WO99062596

(56) Entgegenhaltungen:
- US-A- 4 838 272
- US-A- 5 047 005
- US-A- 5 267 938
- US-A- 5 562 707
- US-A- 5 620 463
- US-A- 5 718 662
- US-A- 5 725 471

## Beschreibung

Die vorliegende Erfindung betrifft eine Vorrichtung zur Stimulation eines Körperteils.

Die Behandlung von zentralbedingten Lähmungen gehört in der medizinischen Forschung zu einem der zentralen Problemfelder. Zentrale Lähmungen können aufgrund von Himschädigungen oder Rückenmarksverletzungen entstehen. Sie sind häufig Folge von Schlaganfällen, von angeborenen Hirnschäden, von Hirntumoren oder von äußeren Verletzungen. Zentrale Lähmungen sind oftmals mit schmerzhaften spastischen Muskelverkrampfungen verbunden. Sie sind derzeit weder operativ noch medikamentös ausreichend behebbar.

Zentrale Lähmungen können derzeit lediglich durch konventionelle Krankengymnastik oder durch Aktivierung von Nerven oder Muskeln des gelähmten Körperteils durch elektrische Reize behandelt werden. Dazu werden Elektroden auf oder unter der Haut des gelähmten Körperteils angebracht, so daß ein elektrisches Feld im Bereich der zu aktivierenden Nerven oder Muskeln des gelähmten Körperteils erzeugt wird. Dies kann nur zu einer begrenzten Reaktivierung der Muskeln führen. Eine dauerhafte Rehabilitation und damit Heilung der Lähmung kann damit jedoch nicht erreicht werden. Bei dieser Behandlung treten außerdem häufig erhebliche Schmerzen auf.

Aus der amerikanischen Patentschrift 5,620,463 ist ein elektrophysiologisches Konditionierungssystem bekannt, welches Konditionierungsapplikatoren aufweist, die elektromagnetische Konditionierungssignale übertragen, welche dazu geeignet sind, grundlegende physiologische Effekte, wie die Entspannung des Nervensystems, die Stimulation der Blutzirkulation und die Stimulation von normaler Zellreparatur und Regeneration herbeizuführen und dazu geeignet sind, den natürlichen Selbstschutz und Heilungsmechanismus von Menschen und Tieren zu steigern. Dazu werden Magnetisierungsspulen verwendet, die ein magnetisches Feld erzeugen, wenn ein Strompuls sich durch die Spule entlädt. Ein solches Konditionierungssystem ist allerdings nicht für die Behandlung von zentralen Lähmungen geeignet.

Aufgabe der vorliegenden Erfindung ist die Bereitstellung einer Vorrichtung zur Behandlung von zentralen Lähmungen derart, daß ein dauerhafter Rehabilitationseffekt herbeigeführt wird.

Erfindungsgemäß wird eine Vorrichtung zur Stimulation eines gelähmten Körperteils zur Verfügung gestellt, mit deren Hilfe eine glatte und schmerzfreie zusammengesetzte und koordinierte Bewegung des betreffenden Körperteils hervorgerufen werden kann. Der durch die Lähmung bedingte Ausfall der propriozeptiven Afferenzen (Biosensoren, z.B. Muskelspindeln) soll soweit wie möglich ersetzt werden, um durch eine Neuromodulation die plastischen Fähigkeiten des Zentralnervensystems so früh wie möglich anzuregen.

Die erfindungsgemäße Vorrichtung zur Stimulation eines Körperteils weist die merkmale des Anspruchs 1 auf.

Vorzugsweise ist das Stimulationsmuster der Regelung nach dem normalphysiologischen Aktivierungsmuster optimiert. Es werden hierbei ermüdungsabhängige Faktoren berücksichtigt, und es findet eine Anpassung an neuronale Erregbarkeiten statt.

Die erfindungsgemäße Vorrichtung erlaubt somit die Erzeugung von genau definierten Bewegungen des zentral gelähmten Körperteils. Diese Bewegungen sind regelmäßig aus mehreren Teilbewegungen zusammengesetzt und natürlichen Bewegungen des Patienten möglichst naturgetreu nachempfunden, zum Beispiel Greifbewegungen oder Gehbewegungen. Durch eine repetitive magnetische Stimulation werden hauptsächlich propriozeptive Afferenzen sowohl adäquat durch die induzierten Bewegungen als auch durch direkte Aktivierung von afferenten Nervenfasern ausgelöst. Eine regelmäßige Wiederholung induzierter Bewegungen kann im Zentralen Nervensystem einen Lemeffekt bewirken, der schließlich dazu führt, daß der Patient mit dem gelähmten Körperteil die induzierten Bewegungen wieder selbständig (aktiv) ausführen kann. Ausgehend von diesem teilrehabilitierten Zustand kann der Patient dann auch andere Bewegungen wieder erlernen.

Dieses Verfahren kann grundsätzlich zur Behandlung beliebiger zentral gelähmter Körperteile eingesetzt werden. Es ist technisch nicht auf den Menschen beschränkt, sondern kann auch bei Tieren, etwa Rennpferden, mit lokalen Lähmungserscheinungen eingesetzt werden.

Die erfindungsgemäße Vorrichtung weist zwei oder mehrere Spulen Sᵢ auf. Vorzugsweise verwendet man drei, vier oder fünf Spulen zur Stimulation des gelähmten Körperteils. Für einfache Bewegungen kann auch eine einzelne Spule verwendet werden. Die Spulen müssen derart beschaffen sein, daß sie über Inervationszonen des gelähmten Körperteils so positioniert werden können, daß dort durch Induktion ein elektrisches Feld entsteht, wenn ein Stromimpuls I (Sᵢ) die Spule Sᵢ durchläuft.

Jede Spule Sᵢ verfügt vorzugsweise über eine Stromversorgung, die die zur Erzeugung von Magnetfeldern notwendigen Stromimpulse I (Sᵢ) erzeugt. Es kann aber auch eine gemeinsame Stromversorgung verwendet werden. Diese Stromversorgungen werden von einem Stromimpulsgenerator gesteuert, der Zeitpunkt, Frequenz, Dauer und Intensität der Stromimpulse I (Sᵢ) vorgibt.

Der Stromimpulsgenerator generiert die Stromimpulse nach vorgegebenen Mustern, die jeweils bestimmten zusammengesetzten, nämlich den physiologischen Bewegungsabläufen des betreffenden Körperteils entsprechen. Dabei kann eine Vielzahl von Mustern in einem Speichermedium aufbewahrt werden, auf das der Stromimpulsgenerator jederzeit zugreifen und das er modifizieren kann.

Die Intensität der Stromimpulse I (Sᵢ) bestimmt die Feldstärke des jeweils erzeugten Magnetfeldes. Die Feldstärke des angewandten Magnetfeldes muß eine bestimmte Schwelle überschreiten, damit eine Bewegung ausgelöst wird. Diese Schwelle kann mit dem betroffenen Körperteil und mit dem Patienten variieren.

Dauer und Frequenz der Stromimpulse beeinflußen in erheblichem Umfang die Ausführung der induzierten Bewegung, das heißt ihre Rundheit oder Eckigkeit. Aber auch im therapeutischen Bereich haben Dauer und Frequenz der Stromimpulse einen großen Einfluß. Vorzugsweise liegt die Impulsfrequenz f (I (Sᵢ)) in einem Bereich von 10 Hz bis 30 Hz, besonders bevorzugt in einem Bereich von 15 Hz bis 25 Hz. Diese Frequenzen liegen im physiologischen Bereich für das Ansteuern der Muskenl. Ein Stromimpuls entspricht hierbei vorzugsweise, aufgrund der energetischen Optimierung, dem Ausschnitt einer Sinusschwingung. Die Sinusschwingung hat dabei, wiederum im Hinblick auf ihre energetische Optimierung, vorzugsweise eine Periodendauer in einem Bereich von 1,19*10⁻⁴ s bis 3,77*10⁻⁴ s, besonders bevorzugt in einem Bereich von 1,19*10⁻⁴ s bis 2,15*10⁻⁴ s. Der Ausschnitt erstreckt sich vorzugsweise von 0 bis zu einem Wert in einem Bereich von 0 bis 2π, besonders bevorzugt bis zum einem Wert von k*π/4, wobei k 1, 2, 3, oder 4 ist. In einer weiteren bevorzugten Ausführungsform wird die Sinusschwingung bei einem Wert in dem Bereich von 0 bis π/4 abgebrochen, so daß sich ein hoher Wert für dI (Sᵢ)/dt ergibt, was eine verbesserte Stimulationswirkung mit sich bringt.

In einer bevorzugten Ausführungsform weist die Vorrichtung zur Steuerung oder Regelung der Stromversorgung(en) für die Spulen Sᵢ mindestens einen Sensor zur Erfassung der momentanen Position des Körperteils auf, um dadurch die Stromversorgung(en) für die Spulen entsprechend steuern bzw. regeln zu können. Vorzugsweise verwendet man hier einen oder mehrere, eventuell auch eine Kombination der folgenden Sensoren: einen Positionsschalter, vorzugsweise einen 3-Punkt-Schalter, ein Winkelpotentiometer, ein Ultraschallmeßsystem oder eine Infrarotkamera. Bei Verwendung von Winkelmeßpotentiometern wählt man vorzugsweise eine Anordnung aus drei Potentiometern, deren Winkelsignale aufaddiert werden. Die einzelnen Potentiometer müssen dadurch nicht exakt an die Gelenkachse angepaßt werden. Bei einem Ultraschallmeßsystem werden Ultraschallsender auf geeigneten Stellen des betreffenden Körperteils befestigt. Die Signale dieser Sender werden von einem ortsfesten Empfänger hinsichtlich ihrer Position erfaßt. Verwendet man Infrarotkameras, so wird durch an geeigneten Stellen des entsprechenden Körperteils befestigte Infrarot-LED aus dem Abbild zweier Kameras die Position zurückgerechnet.

In einer anderen bevorzugten Ausführungsform der Vorrichtung wird die Vorrichtung speziell an einen bestimmten Patienten angepaßt, so daß die Vorrichtung auch in einem "Feed-Forward"-Modus ohne jegliche Sensoren verwendet werden kann.

Vorzugsweise enthält die Vorrichtung zur Steuerung und Regelung der Stromversorgung(en) für die Spulen eine Regelungseinheit, die auf ein Signal reagiert, das mindestens einen Zustandsparameter für mindestens einen Muskel des Körperteils repräsentiert. Der Zustand eines Muskels setzt sich zusammen aus der mechanischen Ausdehnung (elastische und dämpfende Faktoren) und aus der inervatorischen, kontraktilen Muskelaktivierung. Vorzugsweise wird dieses Signal aus einem Elektromyogramm gewonnen, das an mindestens einem Muskel des Körperteils gemessen wird. Die Elektromyographie stellt eine Methode zur Registrierung von Muskelaktionspotentialen dar. Ein Elektromyogramm kann somit Auskunft über induzierte oder willensgesteuerte intendierte Aktionspotentiale geben, durch die die stimulierte Bewegung des betreffenden Muskels unterstützt wird. Es können damit sowohl das Maß der Lähmung und der bereits erzielten Rehabilitation als auch die Ermüdung des Muskels bestimmt werden. Auch der Einfluß der Unterstützung der induzierten Bewegungen durch den Patienten infolge eigener Willensanstrengungen kann mit Hilfe dieses Verfahrens quantitativ oder qualitativ abgeschätzt werden. Diese Informationen ermöglichen eine Anpassung der Stromimpulse I (Sᵢ), das heißt von deren Intensität, Frequenz und Dauer, an die konkrete Behandlungssituation des Patienten. Damit kann die Rehabilitation individualisiert und intensiviert werden.

Die Regelung der Behandlung kann prinzipiell in beliebigen Zeitabständen erfolgen, beispielsweise etwa nach jeweils mehreren Sekunden, was ein ständiges Monitoring der Situation bedeuten würde, oder auch nach jeweils mehreren Tagen oder Wochen, was einer Grobkontrolle der Rehabilitationsschritte gleichkommen würde.

In einer weiteren bevorzugten Ausführungsform der Erfindung weist die Vorrichtung zur Steuerung oder Regelung der Stromversorgung(en) für die Spulen Sᵢ mindestens einen Sensor zur Erfassung von auf das entsprechende Körperteil wirkenden Kräften auf, um damit eine angepaßte Steuerung bzw. Regelung der Stromversorgung(en) für die Spulen zu ermöglichen. Vorzugsweise handelt es sich bei diesem Sensor um einen druckabhängigen Widerstand. Dazu führt man beispielsweise eine piezoelektrische Kapazitätsmessung aus, oder man verwendet Dehnungsmeßstreifen.

Vorzugsweise ist in der Vorrichtung zur Steuerung oder Regelung der Stromversorgung(en) für die Spulen ein Lemalgorithmus integriert. Während einer Stimulation werden hier Stimulationsauswirkung und Effekt beobachtet, analysiert und in einer Speichereinheit aufgenommen. Dadurch läßt sich der Stimulationseffekt in darauffolgenden Zyklen patientenspezifisch optimieren. Alternativ dazu kann die Vorgehensweise auch dergestalt sein, daß die Bewegung zunächst gesteuert ausgeführt wird, nach Beendigung der Bewegung die Ist-Position des Körperteils mit dessen Soll-Position verglichen wird und sodann die Steuerparameter so geändert werden, daß das Ziel bei dem nächsten Stimulationsdurchgang noch besser erreicht wird. Diese Art der "Feed-Forward"-Regelung entspricht eher den physiologischen Gegebenheiten. Realisiert werden kann diese Regelung beispielsweise durch sogenannte Neuro-Controller (neuronale Netze) oder durch eine adaptive Regeleinrichtung. In einer weiteren Nutzungsmöglichkeit für den Lemalgorithmus wird das Stimulationsmuster an die physiologische Signalgebung angepaßt.

Weitere Vorteile, Merkmale und Anwendungsmöglichkeiten der Erfindung ergeben sich aus der nachfolgenden Beschreibung eines Ausführungsbeispiels in Verbindung mit der Zeichnung. Es zeigt:
- Fig. 1: Funktionsschaubild einer erfindungsgemäßen Vorrichtung, wobei das zu stimulierende Körperteil ein menschlicher Arm ist.
- Fig. 2: Flußdiagramm der erfindungsgemäßen Stimulation eines Muskels und dadurch hervorgerufener Körperreaktionen.
- Fig. 3: Diagramm des zeitlichen Verlaufs, der durch einen erfindungsgemäß stimulierten Muskel ausgeübten Kraft, unter der Berücksichtigung der Nebensysteme.
- Fig. 4: Diagramm des zeitlichen Verlaufs, der durch einen erfindungsgemäß stimulierten Muskel ausgeübten Kraft, aufgrund der Stimulation.
- Fig. 5: Diagramm des zeitlichen Verlaufs, der durch den Antagonisten ausgeübten Kraft.
- Fig. 6: Funktionsschaubild einer erfindungsgemäßen Vorrichtung mit Peripheriegeräten.

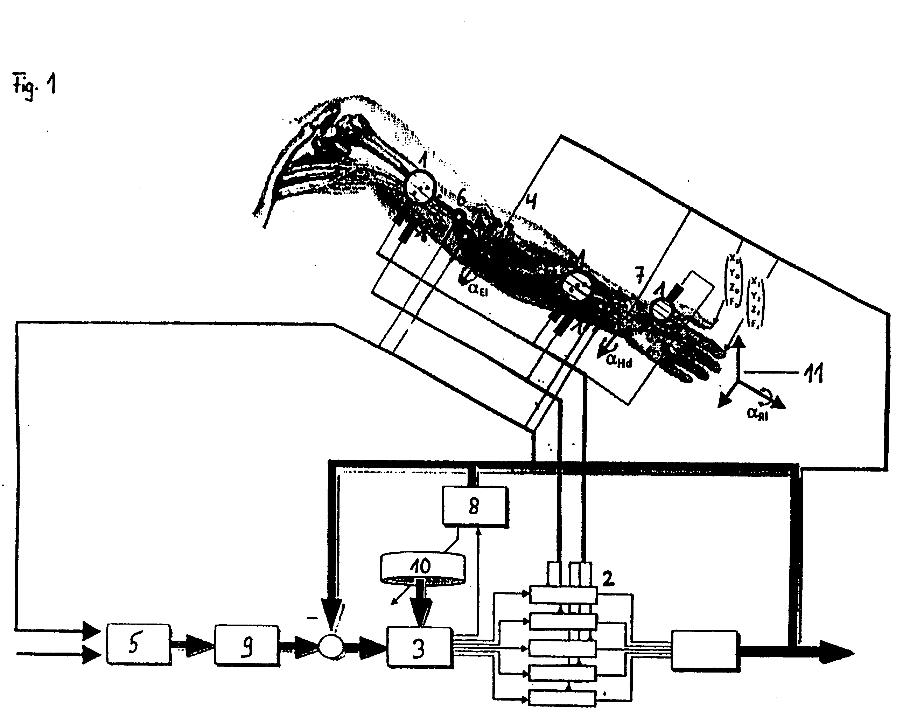

Figur 1 stellt ein Funktionsschaubild einer erfindungsgemäßen Vorrichtung dar. Die hier dargestellte Vorrichtung dient der Stimulation bzw. Neurostimulation eines menschlichen Armes. Fünf Spulen 1 sind am Arm derart angeordnet, daß sie im stromdurchflossenen Zustand an Inervationszonen eines entsprechenden Muskels bzw. einer entsprechenden Muskelgruppe des Arms Magnetfelder erzeugen können. Die fünf Stromversorgungen 2 für die fünf Spulen 1 werden über einen Stromimpulsgenerator 3 angesteuert. Der Stromimpulsgenerator 3 ist für die Generierung eines entsprechenden Stromimpulsmusters verantwortlich, das nötig ist, um eine Stimulation einzelner Muskeln oder Muskelgruppen zu erzielen, was letztlich Schritt für Schritt zu einer bestimmten ausgewählten Bewegung des Armes führt, z.B. zum Anwinkeln des Armes. Die Stimulation der Muskeln bzw. Muskelgruppen muß dabei so erfolgen, daß die Muskeln bzw. Muskelgruppen koordiniert kontrahiert oder dekontrahiert werden. Zwei Spulen 1 sollen dabei regelmäßig nicht gleichzeitig "betrieben" werden, d.h. mit Strom versorgt werden, um ein ungünstiges Zusammenspiel der entsprechenden Magnetfelder zu verhindern, insbesondere um eine positive Überlagerung der Magnetfelder und damit einhergehende enorme Feldstärken zu vermeiden.

Bevor im Stromimpulsgenerator 3 ein Impulsmuster generiert werden kann, wird dem System über eine Regelungseinheit 5 ein bestimmtes Kommando zugeführt. Dies kann entweder ein externer Befehl zur Durchführung einer speziellen Bewegung oder eine willentliche Aktivierung (Intention) der betreffenden Person für eine bestimmte Bewegung sein. Letztere wird durch ein Elektromyogramm 6 entsprechender Muskeln gemessen. Nach Aufnahme eines Kommandos wird dieses Kommando in einem ersten Schritt durch die Regeleinheit 5 in systemverständliche Einzelbefehle umgesetzt. In einer weiteren nachgeschalteten Regeleinheit 9 werden diese Einzelbefehle in Bewegungsabschnitte mit zugehöriger Bewegungs- und Kraftverlaufstrajektorie für die Bewegung des Armes und der Hand umgesetzt. Eine Bewegungs- und Kraftverlaufstrajektorie setzt sich aus einer Mehrzahl von Durchgangspunkten zusammen, wobei sich jeder Durchgangspunkt aus den Gelenkwinkel- und Fingerkraftvorgaben für Arm und Hand zusammensetzt. Im Stromimpulsgenerator 3 werden durch Vergleich zwischen Soll- und Ist-Position die Stromimpulsmuster generiert, die durch die Stromversorgungen 2 für die Spulen 1 jeweils an diese abgegeben werden müssen, um die entsprechenden Muskeln bzw. Muskelgruppen zu stimulieren. Der Stromimpulsgenerator 3 greift zur Generierung des Impulsmusters auf eine in der Vorrichtung integrierte Speichereinheit 10 zu, in der körperteil- bzw. patientenspezifische Informationen gespeichert sind. Mit Hilfe dieser Informationen kann somit das Stromimpulsmuster individuell angepaßt und optimiert werden. Während einer Stimulation werden mittels eines Lemalgorithmus 8 Stimulationsauswirkung und Effekt beobachtet und analysiert, um dann für den darauffolgenden Zyklus optimiert zu werden.

Über Sensoren 4, 7 und 11, beispielsweise Winkelpotentiometer mit Druckerfassung über druckabhängige Widerstände, werden die Gelenkwinkelpositionen und Kräfte auf Daumen und Zeigefinger erfaßt und rückgemeldet. Die Rückmeldung dient zur Regelung der für den darauffolgenden Bewegungsschritt notwendigen Stromimpulse, die von den Stromversorgungen 2 an die Spulen 1 abgegeben werden sollen. Durch die Messung der Kräfte an Daumen und Zeigefinger wird ein geregeltes Greifen von Objekten bzw. ein kraftgeregelter Zusammenschluß von Daumen und Zeigefinger zum Präzisionsgriff ermöglicht.

Dieser Zyklus wird so oft durchlaufen, bis die vorgegebene Bewegung vollendet ist, was ebenfalls durch die Sensoren 4 und 7 festgestellt und gemeldet wird.

Die Magnetstimulation eines Muskels infolge eines Stromimpulses und deren Auswirkung auf den stimulierten Muskel und des übrigen Organismus wird anhand der Figur 2 verdeutlicht. Die Stimulation und ihre Auswirkungen kann in drei Systeme A, B und C unterteilt werden.

In dem Hauptsystem A wird auf der Grundlage der Soll-Koordinaten und der Ist-Koordinaten der Gliedrnaße durch den Stromimpulsgenerator 3 (vgl. auch Figur 1) ein Impuls über die betreffende Stromversorgung 2 an die betreffende Spule 1 zur Stimulation eines Muskels 12 der Gliedmaße ausgegeben. Diese Stimulation wirkt auf den zu stimulierenden Muskel 12 und verursacht dadurch eine Bewegung der Gliedmaße. Die neuen Koordinaten der Gliedmaße nach der Bewegung werden gemessen und an den Stromimpulsgenerator 3 als neue Ist-Koordinaten geleitet. Die von dem stimulierten Muskel 12 im statischen Fall auf einen Kraftmesser, etwa einen druckabhängigen Widerstand, und im dynamischen Fall auf einen Beschleunigungsmesser ausgeübte Kraft wird mit F_{M1}(t) bezeichnet.

Neben diesem Hauptsystem A wird die Bewegung der Gliedmaße allerdings noch durch zwei Nebensysteme B und C beeinflußt. Im antagonistischen Nebensystem B wird durch die Bewegung des stimulierten Muskels 12 eine Bewegung des Antagonisten 13, also des Gegenmuskels zu dem stimulierten Muskel 12, initiiert. Wenn sich der stimulierte Muskel 12 kontrahiert, dehnt sich der Antagonist 13 und bremst oder, richtiger gesagt, balanciert dabei die Bewegung des stimulierten Muskels 12. Dies geschieht durch eine Aktivierung der Muskelspindel 15 des Antagonisten 13, wobei diese Aktivierung über das Rückenmark 14, gesteuert durch das Zentrale Nervensystem 18, reflektorisch zu einer Kontraktion des Antagonisten 13 führt. Die von dem Antagonisten 13 ausgeübte Kraft wird mit F_{M2}(t) bezeichnet.

Bei dem zweiten Nebensystem C handelt es sich um die reflektorische Kopplung des stimulierten Muskels 12 selbst. Durch die Bewegung des stimulierten Muskels 12 wird über dessen Muskelspindel 16 und über das Rückenmark 17, gesteuert durch das Zentrale Nervensystem 18, die Stimulation dieses Muskels 12 direkt beeinflußt. Diese Kopplung des stimulierten Muskels 12 ist in der Kraft F_{M1}(t) enthalten. Allerdings trägt die Kopplung nur zeitverzögert zu der Kontraktion des stimulierten Muskels 12 bei, so daß ihr Einfluß auf den Verlauf der Kraft F_{M1}(t) nicht konstant ist. Während bei Gesunden diese Nebensysteme von untergeordneter Bedeutung sind, sind bei Kranken mit cerebralen Lähmungen diese Nebensysteme, durch die Enthemmung der Reflexe, starke Störfaktoren für die Kraftentwicklung des stimulierten Muskels.

Die beiden Nebensysteme müssen neben dem Hauptsystem bei der Stimulation des Muskels berücksichtigt werden. Die Bewegung der Gliedmaße folgt nämlich der Gesamtkraft F_{G}(t), die die Summe aus den Einzelkräften F_{M1}(t) und F_{M2}(t) darstellt. Um den Effekt der Stimulierung steuern zu können, muß die Auswirkung einer Änderung der Stimulation auf die Bewegung der Gliedmaße ermittelt werden. Dies erfordert eine Trennung der Effekte der Stimulation von den Effekten der Aktivierung des Antagonisten und der Kopplung. Hierzu werden von dem Stromimpulsgenerator Einzelimpulse in einem Abstand von mehr als 5 Sekunden mit steigender Amplitude abgegeben. Dies entspricht gewichteten Dirac-Impulsen. Hierdurch kann die Systemantwort des Hauptsystems des Muskels unter den vorgegebenen Bedingungen bestimmt werden.

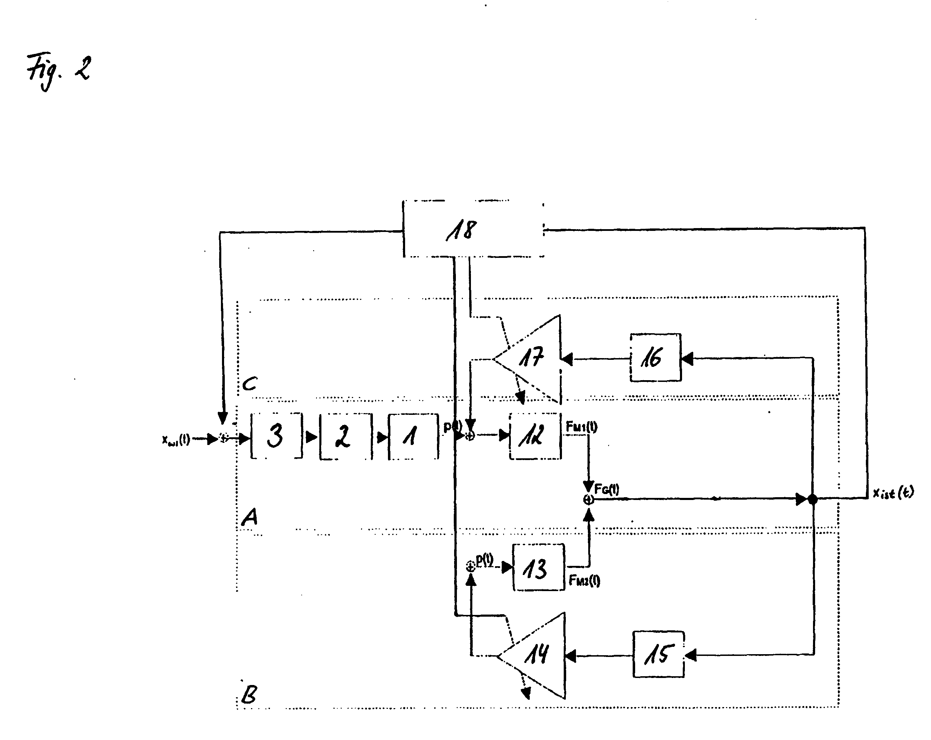

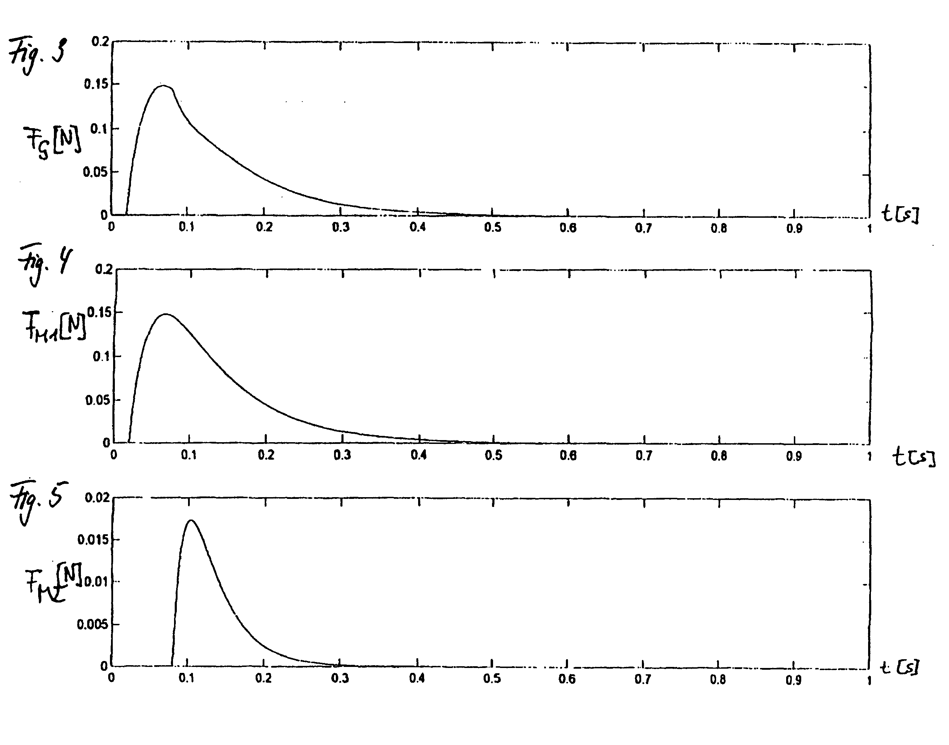

Figur 4 zeigt ein Beispiel für den zeitlichen Verlauf der Kraft F_{M1}(t). Dieser Verlauf kann durch die Exponentialfunktion A*(exp(-t/T1)-exp(-t/T2)) beschrieben werden. Im idealen Fall ist dieser Kraftverlauf gleich dem der Gesamtkraft F_{G}(t). Der tatsächliche in Figur 3 dargestellte Verlauf der Gesamtkraft unterscheidet sich allerdings von dem idealen Verlauf durch einen stärkeren Abfall nach dem Maximum. Dieser Unterschied ist bei Gesunden relativ gering, bei Kranken mit cerebralen Lähmungen allerdings von großer Bedeutung. Dieser Verlauf der Gesamtkraft F_{G}(t) kann durch die oben beschriebene Exponentialfunktion angenähert werden. Durch dieses Verfahren wird der Kraftverlauf F_{M1}(t) bestimmt. Wie in Figur 2 zu sehen ist entsteht der Verlauf der Gesamtkraft F_{G}(t) durch eine Überlagerung der Kräfte F_{M1}(t) und F_{M2}(t). Daraus ergibt sich, daß die Differenz aus dem Verlauf der Gesamtkraft F_{G}(t) und der Näherung durch die Exponentialfunktion F_{M1}(t) den Verlauf der Kraft des zweiten Muskels F_{M2}(t) aufgrund des antagonistischen Nebensystems darstellt. Der Verlauf, angenähert durch die Exponentialfunktion, ist in Figur 5 dargestellt. Somit kann der Einfluß des Antagonisten auf die stimulierte Kontraktion bestimmt werden, während die Kopplung des stimulierten Muskels nicht separiert werden kann.

Aufgrund des nun bestimmten Stimulationssystems können die Startwerte für die Stimulation bestimmt werden. Damit kann die Reaktion des stimulierten Muskels bei der Anwendung repetitiver Impulse vorhergesagt und aufgrund dessen die Wirkung der Stimulation beurteilt werden. Während der Stimulation läßt allerdings die Reaktion des Antagonisten deutlich nach. Um eine langsame, gedämpfte und kontrollierte Bewegung der stimulierten Gliedmaße zu erreichen, wird deshalb der Einfluß des Antagonisten auch während der Stimulation iterativ bzw. adaptiv im closed loop bestimmt und berücksichtigt.

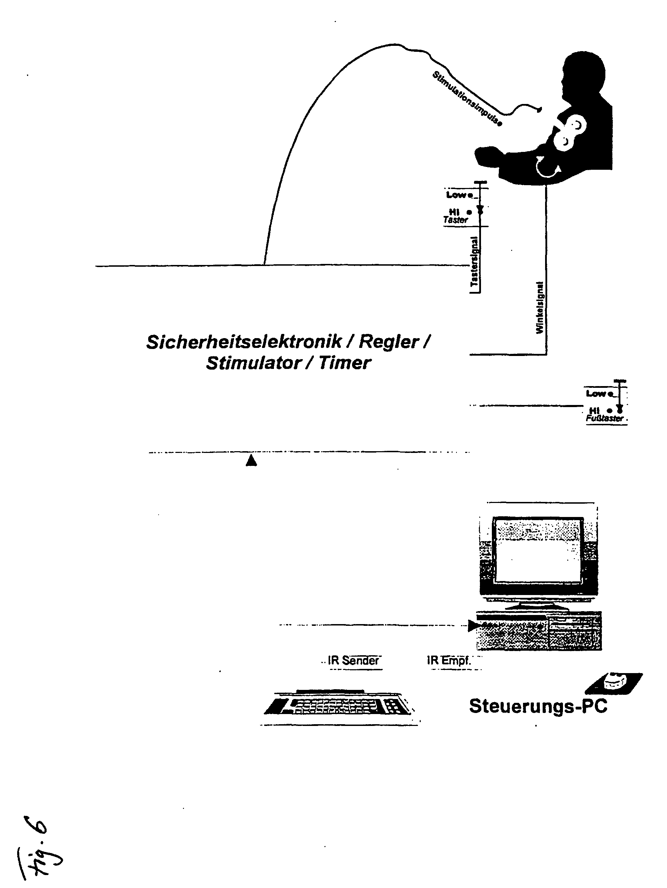

Vorzugsweise umfaßt die erfindungsgemäße Vorrichtung und das erfindungsgemäße Verfahren ein Sicherheitssystem. Dieses Sicherheitssystem verhindert, daß die Stimulation in unbeabsichtigter Weise stattfindet. Figur 6 zeigt, daß dieses Sicherheitssystem unter anderem zwei Taster aufweist. Den ersten Taster hält der Patient während der Stimulation gedrückt. Dies ermöglicht dem Patienten die Stimulation schnellstmöglich zu beenden. Bei dem zweiten Taster handelt es sich um einen Fußtaster, mit dem die Anwesenheit eines Überwachers sichergestellt wird.

Sowohl wenn der Patient, als auch wenn der Überwacher das Drücken des Tasters unterbricht, kann in dieser Zeit keine Stimulation erfolgen.

In Figur 6 ist auch zu sehen, daß vorzugsweise die Steuerungseinheit des Stimulators über eine nicht leitende Verbindung, in diesem Fall eine Infrarotverbindung, mit dem Stimulator kommuniziert. Damit wird eine galvanische Verbindung zwischen dem Bediener, dem Patienten und der Stimulationseinheit verhindert. Dies ist besonders dann von Vorteil, wenn eine galvanische Verbindung zwischen dem Patienten und Erde unterbunden werden soll, was im Hinblick auf die möglicherweise durch die Magnetfelder der Spulen in dem Patienten oder seiner unmittelbaren Umgebung, etwa dem Behandlungsstuhl, induzierten Strömen ratsam ist. Insbesondere im Falle einer fehlerhaft funktionierenden Spule ist dies wichtig.

Zusätzlich zu diesen Sicherheitseinrichtungen, sorgen noch einige Kontrollmechanismen für eine planmäßige Stimulation des Patienten. Durch eine Timerfunktion, die mit den einzelnen Stimulatoren in Verbindung steht, wird zum Beispiel verhindert, daß nie mehrere Stimulatoren gleichzeitig im Betrieb sein können. Damit kann die gegenseitige Einwirkung von Spulen aufeinander verhindert werden, die zu ungewollten Kraftübertragungen auf den Patienten und damit zu körperlichen Schädigungen des Patienten führen können.

## Patentansprüche

1. Vorrichtung zur Stimulation eines Körperteils, die mindestens folgende Elemente aufweist:
a) Mindestens zwei Spulen Sᵢ (1) mit mindestens einer Stromversorgung (2), wobei die mindestens zwei Spulen Sᵢ derart beschaffen sind, dass sie über Inervationszonen des Körperteils, insbesondere an Endaufzweigungen motorischer Nervenfasern oder peripherer Nerven, so positioniert werden können, dass dort ein Magnetfeld entsteht, das eine bestimmte schwelle überschreitet, damit eine Bewegung ausgelöst wird, und
b) eine Vorrichtung (5, 9) zur Steuerung oder Regelung der mindestens einen Stromversorgung (2) für die Spulen S; (1), mit einer Timerfunktion, die verhindert, daß zwei spulen gleichzeitig mit strom versorgt werden wobei die vorrichtung zur Steuerung oder Regelung mindestens folgendes Element aufweist:
einen Stromimpulsgenerator (3) der derart beschaffen ist, dass er Stromimpulse I (Sᵢ) mit Impulsfrequenzen f (I (Sᵢ)) und Impulsdauern d (I (Sᵢ)) durch die mindestens eine Stromversorgung (2) an die Spulen Sᵢ(1) nach vorgegebenen Mustern abgibt, die jeweils bestimmten physiologischen Bewegungsabläufen des Körperteils entsprechen, so daß Muskeln (15) des Körperteils koordiniert kontrahiert oder dekontrahiert werden, wodurch sich eine koordinierte zusammengesetzte Bewegung des Körperteils ergibt.

2. Vorrichtung nach Anspruch 1, **dadurch gekennzeichnet, daß** die Vorrichtung (5, 9) zur Steuerung oder Regelung der mindestens einen Stromversorgung (2) für die Spulen Sᵢ (1) mindestens einen Sensor (4, 7, 11) zur Erfassung der Position des Körperteils zur Steuerung bzw. Regelung der mindestens einen Stromversorgung (2) für die Spulen Sᵢ (1) aufweist.

3. Vorrichtung nach einem der vorhergehenden Ansprüche, **dadurch gekennzeichnet, daß** die Vorrichtung (5, 9) zur Steuerung oder Regelung der mindestens einen Stromversorgung (2) für die Spulen Sᵢ (1) eine Regelungseinheit enthält, die auf ein Signal reagiert, das mindestens einen Zustandsparameter für mindestens einen Muskel (15) des Körperteils repräsentiert.

4. Vorrichtung nach Anspruch 3, **dadurch gekennzeichnet, daß** das Signal aus einem an mindestens einem Muskel (15) des Körperteils gemessenen Elektromyogramm (EMG) (6) gewonnen wird.

5. Vorrichtung nach einem der vorhergehenden Ansprüche, **dadurch gekennzeichnet, daß** die Vorrichtung (5, 9) zur Steuerung oder Regelung der mindestens einen Stromversorgung (2) für die Spulen Sᵢ (1) mindestens einen Sensor (4, 7, 11) zur Erfassung von auf das Körperteil wirkenden Kräften zur Steuerung bzw. Regelung der mindestens einen Stromversorgung (2) für die Spulen Sᵢ (1) aufweist.

6. Vorrichtung nach Anspruch 5, **dadurch gekennzeichnet, daß** mindestens ein Sensor (4, 7, 11) ein druckabhängiger Widerstand ist.

7. Vorrichtung nach einem der vorhergehenden Ansprüche, **dadurch gekennzeichnet, daß** die Vorrichtung (5, 9) zur Steuerung oder Regelung der mindestens einen Stromversorgung (2) für die Spulen Sᵢ (1) einen Lernalgorithmus (8) enthält, der auf der Basis von Informationen über die durchgeführten Bewegungen des Körperteils und über den Zustand des Körperteils die Steuerung oder Regelung der mindestens einen Stromversorgung (2) für die Spulen Sᵢ (1) beeinflusst.

8. Vorrichtung nach einem der vorhergehenden Ansprüche, **dadurch gekennzeichnet, daß** mindestens eine Impulsfrequenz f(I (Sᵢ)) im Bereich von 10 Hz bis 30 Hz, vorzugsweise von 15 Hz bis 25 Hz liegt.

9. Vorrichtung nach einem der vorhergehenden Ansprüche, **dadurch gekennzeichnet, daß** mindestens ein Stromimpuls I (Sᵢ) einem Ausschnitt einer Sinusschwingung entspricht.

10. Vorrichtung nach Anspruch 9, **dadurch gekennzeichnet, daß** der Ausschnitt der Sinusschwingung sich von Null bis zu einem Wert in einem Bereich von 0 bis 2π erstreckt, vorzugsweise bis zu einem Wert von k*π/4, wobei k 1, 2, 3 oder 4 ist.

## Claims

1. Device for the stimulation of a body part comprising at least the following elements:
a) at least two coils Sᵢ (1) with at least one power supply (2) wherein the at least two coils are provided in such a way that they can be arranged at innervation zones of the body part, in particular at end branches of motor nerve fibres or peripheral nerves for the generation of a magnetic field, wherein the magnetic field exceeds a certain threshold in order for a movement to be initiated, and
b) a device (5, 9) for the open-loop or closed-loop control of the at least one power supply (2) for the coils Sᵢ (1), having a timer function which prevents that two coils are supplied with current simultaneously, wherein the device for the open-loop or closed-loop control comprises at least the following element:
a current pulse generator (3) for the emission of current pulses I (S;) at pulse frequencies f (I(Sᵢ)) and pulse durations d (I(Sᵢ)) through the at least one power supply (2) to the coils Sᵢ (1) in accordance with predetermined patterns which correspond to typical physiological movements of the body part respectively, so that muscles (15) of the body part are contracted or decontracted in a coordinated manner, so that a coordinated composite movement of the body part is obtained.

2. Device according to Claim 1, **characterized in that** the device (5, 9) for the open-loop or closed-loop control of the at least one power supply (2) for the coils Sᵢ (1) has at least one sensor (4, 7, 11) for sensing the position of the body part for controlling or regulating the at least one power supply (2) for the coils Sᵢ (1).

3. Device according to one of the preceding claims, **characterized in that** the device (5, 9) for the open-loop or closed-loop control of the at least one power supply (2) for the coils Sᵢ (1) includes a closed-loop control unit which responds to a signal which represents at least one state parameter for at least one muscle (15) of the body part.

4. Device according to Claim 3, **characterized in that** the signal is obtained from an electromyogram (EMG) (6) measured at at least one muscle (15) of the body part.

5. Device according to one of the preceding claims, **characterized in that** the device (5, 9) for the open-loop or closed-loop control of the at least one power supply (2) for the coils Sᵢ (1) has at least one sensor (4, 7, 11) for sensing forces acting on the body part for controlling or regulating the at least one power supply (2) for the coils Sᵢ (1).

6. Device according to Claim 5, **characterized in that** at least one sensor (4, 7, 11) is a pressure-dependent resistor.

7. Device according to one of the preceding claims, **characterized in that** the device (5, 9) for the open-loop or closed-loop control of the at least one power supply (2) for the coils Sᵢ (1) includes a learning algorithm (8), which influences the controlling or regulating of the at least one power supply (2) for the coils Sᵢ (1) on the basis of information on the movements of the body part carried out and on the state of the body part.

8. Device according to one of the preceding claims, **characterized in that** at least one pulse frequency f (I(Sᵢ)) lies in the range from 10 Hz to 30 Hz, preferably from 15 Hz to 25 Hz.

9. Device according to one of the preceding claims, **characterized in that** at least one current pulse I (Sᵢ) corresponds to a segment of a sinusoidal oscillation.

10. Device according to Claim 9, **characterized in that** the segment of the sinusoidal oscillation extends from zero to a value in a range from 0 to 2π, preferably to a value of k*π/4, where k is 1, 2, 3 or 4.

## Revendications

1. Dispositif de stimulation d'une partie du corps comportant au moins les éléments suivants :
a) deux ou plus de deux bobines Sᵢ (1) comportant au moins une alimentation en courant (2), les deux ou plus de deux bobines Sᵢ étant constituées de telle sorte qu'elles puissent être placées sur des zones innervées de la partie du corps, notamment au niveau de terminaisons de fibres nerveuses motrices ou de nerfs périphériques, et ce, de manière qu'il s'y produise un champ magnétique qui dépasse un seuil déterminé afin qu'un mouvement se déclenche, et
b) un dispositif (5, 9) de commande ou de réglage de la ou des alimentations en courant (2) des bobines Sᵢ (1) lequel comporte une fonction de rythmeur empêchant que deux bobines soient simultanément approvisionnées en courant, le dispositif de commande ou de réglage comportant au moins l'élément suivant :
un générateur d'impulsion de courant (3) constitué de telle sorte qu'il délivre aux bobines Sᵢ (1), par l'intermédiaire de la ou des alimentations en courant (2), des impulsions de courant I (Sᵢ) ayant des fréquences d'impulsion f (I (Sᵢ)) et des durées d'impulsion d (I (Sᵢ)) selon des modèles prescrits qui correspondent chacun à des déroulements de mouvement physiologique déterminés de la partie du corps de sorte que des muscles (15) de la partie du corps soient contractés ou relâchés de manière coordonnée et produisent ainsi un mouvement composé coordonné de la partie du corps.

2. Dispositif selon la revendication 1, **caractérisé en ce que** le dispositif (5, 9) de commande ou de réglage de la ou des alimentations en courant (2) des bobines Sᵢ (1) comporte au moins un capteur (4, 7, 11) de détection de la position de la partie du corps servant à commander ou à régler la ou les alimentations en courant (2) des bobines Sᵢ (1).

3. Dispositif selon l'une des revendications précédentes, **caractérisé en ce que** le dispositif (5, 9) de commande ou de réglage de la ou des alimentations en courant (2) des bobines Sᵢ (1) contient une unité de réglage qui réagit à un signal représentant au moins un paramètre d'état d'au moins un muscle (15) de la partie du corps.

4. Dispositif selon la revendication 3, **caractérisé en ce que** le signal est obtenu à partir d'un électromyogramme (EMG) (6) mesuré au niveau d'au moins un muscle (15) de la partie du corps.

5. Dispositif selon l'une des revendications précédentes, **caractérisé en ce que** le dispositif (5, 9) de commande ou de réglage de la ou des alimentations en courant (2) des bobines Sᵢ (1) comporte au moins un capteur (4, 7, 11) de détection des forces agissant sur la partie du corps pour commander ou régler la ou les alimentations en courant (2) des bobines Sᵢ (1).

6. Dispositif selon la revendication 5, **caractérisé en ce qu'**au moins un capteur (4, 7, 11) est une résistance fonction de la pression.

7. Dispositif selon l'une des revendications précédentes, **caractérisé en ce que** le dispositif (5, 9) de commande ou de réglage de la ou des alimentations en courant (2) des bobines Sᵢ (1) contient un algorithme d'apprentissage (8) qui influe sur la commande ou le réglage de la ou des alimentations en courant (2) des bobines Sᵢ (1) en se fondant sur des informations relatives aux mouvements exécutés par la partie du corps et à l'état de la partie du corps.

8. Dispositif selon l'une des revendications précédentes, **caractérisé en ce qu'**au moins une fréquence d'impulsion f (I (Sᵢ)) est comprise entre 10 Hz et 30 Hz, de préférence entre 15 Hz et 25 Hz.

9. Dispositif selon l'une des revendications précédentes, **caractérisé en ce qu'**au moins une impulsion de courant I (Sᵢ) correspond à une partie d'une oscillation sinusoïdale.

10. Dispositif selon la revendication 9, **caractérisé en ce que** la partie de l'oscillation sinusoïdale s'étend depuis zéro jusqu'à une valeur comprise entre 0 et 2π, de préférence jusqu'à une valeur k * π / 4, k étant égal à 1, 2, 3 ou 4.
